# EUROPEAN PATENT APPLICATION

(11) **EP 3 248 591 A1**
(43) Date of publication of application: **29.11.2017**
(21) Application number: 16171027.2
(22) Date of filing: 24.05.2016
(51) Int. Cl.: A61K 8/64, A61Q 11/00, A61K 6/00, C07K 7/00, A61K 38/04, A61P 1/02

(54) **METHOD AND PRODUCT FOR CARIES TREATMENT**

(71) Applicant: Credentis AG, 5210 Windisch (CH)
(72) Inventor: Hug, Michael, 4800 Zofingen (CH); Lysek, Dominikus Amadeus, 5210 Windisch (CH)
(74) Representative: Moré, Solveig Helga

(57) **Abstract**

The present invention provides new dental care products comprising self-assembling peptides (SAP) that are capable of undergoing self-assembly at a certain pH for use in dental care, e.g. preventing and/or treating a tooth lesion such as a caries lesion, remineralising a tooth surface, increasing smoothness or shine or increasing hardness of a tooth surface. The dental care products may be administered following a simple protocol involving multiple steps, thereby enabling non-targeted treatment of a plurality of teeth in a subject, independent of the diagnosis of active caries. The invention also relates to said method as well as kits and devices suitable therefor.

## Description

The present invention provides new dental care products comprising self-assembling peptides (SAP) that are capable of undergoing self-assembly at a certain pH for use in dental care, e.g. preventing and/or treating a tooth lesion such as a caries lesion, remineralising a tooth surface, increasing smoothness or shine or increasing hardness of a tooth surface. The dental care products may be administered following a simple protocol involving multiple steps, thereby enabling non-targeted treatment of a plurality of teeth in a subject, independent of the diagnosis of active caries. The invention also relates to said method as well as kits and devices suitable therefor.

To date, tooth remineralisation is achieved mainly by the delivery of calcium and phosphate ions into tooth lesions or cavities. The calcium and phosphate ions are usually included in toothpastes which also contain e.g. abrasives, fluorides, surfactants and other remineralisation agents. The calcium and phosphate ions may be used in various crystalline forms, e.g. as hydroxyapatite-based materials, or as amorphous calcium phosphate, such as in some casein phosphopeptide-based materials.

More recently, an alternative approach to tooth remineralisation has been described, which is based on short rationally designed self-assembling peptides. WO 2004/007532 discloses peptides that are capable of forming three-dimensional scaffolds, thereby promoting nucleation of de-novo calcium phosphate. These artificial peptides assemble to form beta-sheet, tape-like assemblies. The peptide assemblies can switch from a fluid to a nematic, stiffer gel state in response to chemical or physical triggers. The peptides were designed to form assemblies in response to certain pH, ionic strength and/or temperature conditions in the following hierarchical order: tapes, ribbons, fibrils and fibres. Aggeli et al. (2003, J. Am. Chem. Soc. 125, 9619-9628) analyses pH as a trigger of peptide beta-sheet self-assembly.

Several other self-assembling peptides have been described in the prior art. For example, WO 2010/041636 A1 describes a bioadsorbable peptide tissue occluding agent containing an artificial peptide having 8-200 amino acid residues with the hydrophilic amino acids and hydrophopbic amino acids alternately bonded, which self-assembles into a beta-structure at physiological pH. Self-assembling peptides with alternating hydrophobic and hydrophilic residues or stretches which interact with the extracellular matrix are also disclosed in WO 2008/113030 A2. WO 2010/103887 A1 discloses self-assembling peptides, which comprise basic, hydrophobic and acidic amino acids of a specific primary sequence and peptide gels thereof which have high strength.

Another application, WO 2007/000979 A1, describes self-assembling peptides with polar and non-polar amino acids. The peptides are capable of forming a beta-sheet structure in which the non-polar amino acid residues are arranged on one side of the structure in the assembled form. Amphiphilic self-assembling peptides for use as stable macroscopic membranes, which are used in biomaterial applications, such as slow-diffusion drug delivery, are described in US 6,548,630.

EP 2 327 428 A2 refers to a pharmaceutical composition comprising self-assembling peptide nano-fibers, which are complementary to each other, and further comprising at least one cell, for repairing damaged tissue, such as tissue after a myocardial infarction.

The use of self-assembling peptides for the delivery of bioactive agents has been described, for example in US 2008/199431 A1 and in WO 2009/026729 A1. WO 2006/073889 A2 relates to a composition in which human PDGF is bound directly to peptides which assemble into a gel that slowly releases PDGF *in vivo.* WO 2006/047315 A2 proposes the attachment of therapeutic agents to self-assembling peptides using biotin/streptavidin linkages.

Kirkham et al. relates to self-assembling peptide scaffolds promoting enamel remineralisation (2007, Dent. Res. 86(5), 426-430).

To effectively treat tooth lesions, in particular, sub-surface lesions (i.e., an early caries lesion or white spot), the self-assembling peptide needs to be in a monomeric form/ state outside the tooth lesion to enable diffusion into the lesion, and it needs to switch into an assembled form once inside the tooth lesion. If the peptide assembles outside the lesion, it cannot facilitate remineralisation within the lesion, having a low pH and high ionic strength, as the formed three-dimensional structures are too large to diffuse through the pores. Therefore, assembly of the peptide should be prevented until it reaches its site of action.

WO 2014/027012 A1 provides lyophilized solutions comprising self-assembling peptides for targeted treatment of tooth lesions. Since the solution comprising the peptides has to be applied directly onto the surface of the early caries lesion, the application is restricted to professional users, e.g. dentists. Further, pre-conditioning of the tooth to be treated is very complex, including professional cleaning of the tooth in order to remove plaque, food debris and stains as well as treatment with potentially hazardous chemicals such as sodium hypochlorite and phosphoric acid, subsequent rinsing with water and drying the tooth surface. Brunton et al., 2013, Br. Dent. J.215(4): E6 confirms that, before treatment, lesion was cleaned with a prophylaxis paste, treated with etch solution for 30 seconds to open up the pores to the subsurface lesion and subsequently washed and dried. Lyophilised self-assembling peptide in monomeric form was rehydrated with sterile water and a single drop of the resulting solution immediately applied directly to the lesion surface. Moisture control was ensured until the P11-4 solution was no longer visible (approximately two minutes). The subjects were asked not to brush their teeth in the treated quadrant until 4 days after treatment.

However, due to the complex treatment procedure, these solutions are not suitable for home application, and the use is thus restricted to professional dental practitioners. Further, for application of these solutions, pre-conditioning of teeth is required, involving chemicals with potential risk for human health, e.g. sodium hypochlorite and phosphoric acid, which may cause irritation of the mucosa of the respiratory tract after inhalation and irritation of skin after accidental skin contact, bleaching of clothes, and which imply the danger of abuse.

Since there are subjects which, for psychological reasons, avoid or delay visits to the dental practitioner until caries lesions have so widely progressed that the dentist may prefer drilling and filling over the treatment approach using self-assembling peptides, there is a substantial cost for the patient and/or the health system involved.

Accordingly, in light of the state of the art, the inventors solved the problem of providing a dental care product capable of treating caries lesions that is safe and easy to administer following a simple protocol, preferably for over the counter sale or retail, and which is suitable for home application by the patient or consumer in order to treat or prevent caries and/or remineralise tooth surfaces.

This problem is solved by the present invention, in particular, by the claimed subject-matter.

In a first aspect, the present invention provides a dental care product for use in a method of treating or preventing a tooth lesion and/or in remineralising a tooth surface, wherein the dental care product comprises self-assembling peptides and wherein the self-assembling peptide maintains monomeric form after application in step (v) for at least 1 minute, wherein the self-assembling peptides are capable of undergoing self-assembly at a pH below 7.5, wherein the method comprises
(i) removing dental biofilm from a plurality of teeth by a method selected from the group comprising tooth brushing, flossing of teeth, use of an oral irrigator, and rinsing with a salt and detergent solution;
(ii) removing dental pellicle from a plurality of teeth by a method selected from the group comprising tooth brushing, flossing of teeth, use of an oral irrigator, and rinsing with a salt and detergent solution;
(iii) optionally, removing inorganic deposit from a plurality of teeth by a method selected from the group comprising tooth brushing, rinsing with an acidic solution or rinsing with a salt and detergent solution, optionally, using an oral irrigator, and;
(iv) optionally, rinsing of teeth with a solution having a pH of 7 or more before step (v); and
(v) applying the dental care product to a plurality of teeth, wherein the dental care product is preferably applied using an oral irrigator;
   wherein steps (i) (ii) and, if present, (iii) may be carried out sequentially in any order or simultaneously, and wherein step (v) is carried out after the other steps.

Preferably, the dental care product is liquid, e.g. a solution comprising the self-assembling peptides. Liquids are suitable for use in an oral irrigator. Solid or semi-solid dental care products may also be used.

In the method of the present invention, tooth brushing is preferably carried out with a toothpaste or toothgel selected from a sodium bicarbonate toothpaste or tooth gel and an abrasive toothpaste or abrasive tooth gel. Ingredients may comprise, e.g. carbonates, phosphates, in particular, dicalcium phosphate, silicates, acrylates, alumina, and/or biofilm removing agents, e.g. sodium carbonate, surfactants such as SDS and/or phosphate buffer. Tooth brushing with such abrasive toothpastes or abrasive toothgels may not only remove dental pellicle and biofilm, but may also contribute to removing inorganic deposit. Tooth brushing is preferably carried out for 2-3 minutes. An electrical toothbrush may be used. Tooth brushing may be combined with use of an interdental brush to clean interdental spaces.

As used herein, "rinsing of teeth" may involve keeping a sip of the respective solution in the oral cavity for the required time. Optionally, "rinsing of the teeth" may as well imply administering said solution into the oral cavity using a suitable device, e.g. an oral irrigator.

Flossing of teeth includes use of standard floss or abrasive strips to clean interdental spaces from dental biofilm and/or pellicle. Abrasive strips (also designated finishing strips or polishing strips) can remove dental biofilm and/or pellicle and additionally remove inorganic deposits.

Rinsing with a salt and detergent solution, e.g. comprising monovalent, divalent and/or trivalent ions and ionic and/or non-ionic detergent, may remove biofilm as well as pellicle. It may also contribute to removal of inorganic deposits, in particular, if said solution is acidic (e.g., pH of 3-6, preferably, pH 4-6 or pH 4-5). Suitable salts are, e.g., NaCl, KCl, Na₂HPO₄ and KH₂PO₄. For example, 0.8 % NaCl, 0.02 % KCl, 0.142 % Na₂HPO₄ and 0.027 % KH₂PO₄ may be used in combination with a detergent such as SDS (e.g., 0.1-5%). Suitable detergents are, for example, SDS, e.g., at a concentration of 0.1-5 % or Cocoamidopropyl Betain, e.g., at 0.1-5 % or Sulfopon G1216G, e.g., at 0.1-5 %.

Rinsing, e.g., for 10-30 seconds or about 20-30 seconds, with a buffered, acidic solution may remove inorganic deposits. Acid solutions suitable for removing organic deposit in step (iii) may have a pH of 3-6, preferably, pH 4-6 or pH 4-5. A preferred acidic solution has a pH 5. For example, citrate buffers or phosphate-citrate buffers may be used.

Rinsing can be carried out by introducing a mouthful of the solution to the oral cavity and moving it around in the mouth. Typically, the solution for rinsing is then spit out. The process may be repeated. Rinsing may also be carried out by use of an oral irrigator, which improves access to interdental spaces. Rinsing may be carried out, e.g., for about 10 seconds to 5 minutes, preferably, 20 seconds to 1.5 minute or 30 seconds to 1 minute.

Oral irrigators (also designated oral irrigation device or water flossing devices) may also be used for removal of pellicle and/or biofilm by oral irrigation with water, e.g., using micro-droplet techniques and/or high pressure irrigation and/or pulsating irrigation. Use of oral irrigators is particularly advantageous, since oral irrigators are easy to handle and allow effective cleaning of teeth and gums including spaces which are difficult to reach, such as interdental spaces, and thus rather sensitive to caries.

It is possible, but not required that pure water / tap water is applied using oral irrigators. For example, Philips Sonicare AirFloss Ultra®, Waterpik Aquarius Water Flosser ®, or other standard oral irrigators may be used, as well as special irrigators described herein. Oral irrigation may be for about 20 seconds to 5 minutes, preferably, 30 seconds to 3 minute or 40 seconds to 2 minute or 1 seconds to 1.5 minute. Oral irrigators may be used to apply a salt and detergent solution as described herein and/or an acidic solution as described herein.

Removal of inorganic deposits (in particular, dental calculus) in step (iii) is not essential for treatment of active caries lesions, which are typically not covered by dental calculus (Keyes et al., J Oral Biol 3(1):4-7). However, it may otherwise be beneficial, e.g., for remineralizing tooth surfaces.

Optionally, after steps (i) and (ii) and, if present, (iii), teeth are rinsed in step (iv) with a solution having a pH of 7 or more, optionally, of 7.5 or more. Water such as tap water may be used. Step (iv) is particularly advantageous in case any of steps (i) to (iii) involve an acid, a salt and/or a detergent to prevent that the assembly state of the self-assembling peptides is affected, e.g., by the acid conditions. Even without that, rinsing may help to remove loosened biofilm or pellicle from teeth if, e.g., steps (i) and (ii) are carried out by tooth brushing and/or flossing. In comparison with direct use of the dental care product of the invention for a longer time, which is also possible, this step may save self-assembling peptide. However, direct use of the peptide-containing solution reduces complexity of the method and may thus enhance compliance.

In one embodiment, steps (i), (ii) and (v), and, if present, steps (iii) and/or (iv), may be carried out using an oral irrigator. The use of an oral irrigator all steps of the method is preferred.

In the context of the invention, "a" is understood to be "one or more". Accordingly, two or more of the methods described herein for cleaning teeth from biofilm, pellicle and/or inorganic deposits may be used in combination, which is preferred. It is advantageous if at least one of the cleaning methods cleans interdental spaces, in particular, by flossing or oral irrigation.

For example, the method of the invention may comprise tooth brushing followed by using an oral irrigator, wherein the oral irrigator is at least used for step (v), wherein preferably, the oral irrigator is also used for steps (i), (ii), and, optionally, (iii) and/or (iv), most preferably, for all steps (i) to (v). The method of the invention may comprise tooth brushing and flossing followed by using an oral irrigator, wherein the oral irrigator is at least used for step (v), wherein preferably, the oral irrigator is also used for steps (i), (ii), and, optionally, (iii) and/or (iv), most preferably, for all steps (i) to (v). Optionally, the use of an oral irrigator in steps (i), (ii) and, optionally, (iii) involves oral irrigation with a solution comprising water, salt and detergent. Said solution may be acidic.

In a preferred embodiment, the method comprises tooth brushing, e.g., with an abrasive toothpaste, followed by use of an oral irrigator for oral irrigation, e.g., with a salt and detergent solution, and, as a last step, oral irrigation with a dental care product of the invention.

Dental care products of the present invention may be suitable for home application, wherein they are used without prior etching or NaOCl treatment. The inventors found that, nevertheless, treatment of caries lesions and/or remineralization is possible with the treatment regimen of the invention.

In particular, dental care products of the present invention may be used in treating a tooth lesion, preferably a caries lesion, e.g. a subsurface caries lesion. As used herein, a caries "lesion" is a subsurface lesion or a subsurface microlesion in the tooth or the tooth surface that is normally caused by acid formation of the bacteria present in the oral biofilm (ICDAS-II scale 1-4). As used herein, a tooth "cavity" is a hole in the surface of a tooth (ICDAS-II scale ≥5).

Alternatively or additionally, said dental care products may be used in remineralising a tooth surface, e.g. a dental cavity, a white spot lesion or exposed dentin. Preferably, the products of the invention may be used to prevent progression of early caries lesions to cavitated caries lesions.

Bacteria, in particular from the genera *Streptococcus, Lactobacillus* and *Actinomyces*, produce acid by fermentation of carbohydrates that originate from food. The acid formed upon fermentation results in a demineralization of the hard tooth tissues, i.e. the enamel, dentin and cementum. Tooth lesions and cavities may also be the result of a physical trauma. If left untreated, a caries lesion or cavity may lead to an infection of the pulp chamber, which contains blood vessels and nerves, which may ultimately result in tooth loss.

Generally, the lesion or cavity may be present on any tooth, e.g. on the incisors (Dentes incisivi), the canine teeth (Dentes canini), the premolar teeth (Dentes praemolares) and/or the molar teeth (Dentes molares). Similarly, the lesion or cavity may affect any of the surfaces of a tooth, i.e. on labial, mesial, buccal, palatal, proximal, occlusal and/or distal surfaces. For example, reference to "a lesion" includes reference to more than one lesion, in particular, all lesions of the subject.

Products of the invention are intended for use in routine dental care independent of prior diagnosis of active caries, preferably, without prior diagnosis of active caries, more preferably, also without diagnosis of demineralisation or tooth erosion. Diagnosis of active caries, e.g., of white spots which are considered the first stage of caries, or of more progressed stages, is typically carried out by a dental professional. The invention advantageously avoids the need for diagnosis and treatment by professional dental practitioners and enables subjects to effectively treat and prevent tooth lesions on their own. Alternatively, products of the invention may of course also be applied after diagnosis of active caries.

Preferably, the subjects apply the dental care product regularly, so that beginning caries, e.g., white spots or demineralisations can be remineralised by the product of the invention and recently developed caries lesions are effectively treated at an early stage. Thus, dental care products of the invention may be applied at least every 6 months, preferably, at least every 2 months, at least every month, at least every week or daily. The product can be administered for the rest of the lifetime, e.g., from once a day to once every 6 months.

As used herein, "prior diagnosis of active caries" refers to a caries lesion which has been diagnosed by a professional, e.g. a dentist, but which has not yet been treated. In contrast, prior diagnosis of active caries does not refer to a caries lesion which has been successfully treated before the application of the product of the invention.

Upon application to the oral cavity, the peptides remain in a monomeric, non-assembled state for a certain period to ensure distribution of the product to a plurality of teeth and to allow for entry into subsurface lesions, if any are present. In particular, the dental care products of the invention can be used to fill tooth lesions and/or cavities with a network of interconnected peptides that promote the remineralisation of the lesion by deposition of calcium and phosphate ions, which are present, e.g. in the saliva.

The present invention is inter alia based on the insight that by applying a specific method involving few steps and not involving potentially harmful agents such as etching agents or NaOCl, to clean and pre-condition teeth for subsequent treatment, solutions comprising self-assembling peptides are suitable for home application by the patient or costumer without professional assistance, e.g. by a dentist or dental practitioner.

Further, all steps of said method may be carried out using a suitable, easy to handle application device such as an oral irrigator.

In the dental care products of the invention, the self-assembling peptides may be present in a monomeric state for at least 1 minute, preferably at least 2 minutes, more preferably at least 3 minutes after application effected in step (v). Preferably, the self-assembling peptides are present in a monomeric state for the time span for which the dental care product is applied to the teeth. Specifically, the dental care product may be applied for at least 20 seconds, at least 30 seconds, for at least 1 minute, for at least 3 minutes, or for at least 5 minutes.

Application means that a plurality of teeth, or, preferably, all teeth of a subject are contacted with the dental care product in the way this respective type of product is typically used. For example, a mouthwash is typically used to rinse teeth for a time of 20 seconds to 5 minutes, in particular, about 30 seconds to one minute. An oral irrigator is typically used for 20 seconds to 5 minutes, preferably, for 30-90 seconds, in particular, about 1 minute. The dental care product may be used after normal dental care, e.g. in the evening after brushing and, optionally, flossing, teeth.

As used herein, "subject" refers to any subject having teeth, e.g., a mammal such as a human, a dog, a feline such as a cat, a rodent such as a mouse, rat, hamster, guinea pig, a rabbit, a cow, a horse, a camel, a sheep, a goat or another pet, farm or zoo animal having teeth.

The invention also provides a method for treating a tooth lesion, preferably a caries lesion, and/or for remineralising a tooth surface, comprising applying a dental care product comprising self-assembling peptides and wherein the self-assembling peptides maintain monomeric form after application in step (v) for at least 1 minute, wherein the self-assembling peptides are capable of undergoing self-assembly at a pH below 7.5, wherein the method comprises
(i) removing dental biofilm from a plurality of teeth by a method selected from the group comprising tooth brushing, flossing of teeth, use of an oral irrigator, and rinsing with a salt and detergent solution;
(ii) removing dental pellicle from a plurality of teeth by a method selected from the group comprising tooth brushing, flossing of teeth, use of an oral irrigator, and rinsing with a salt and detergent solution;
(iii) optionally, removing inorganic deposit from a plurality of teeth by a method selected from the group comprising tooth brushing, rinsing with an acidic solution or rinsing with a salt and detergent solution, optionally, using an oral irrigator, and;
(iv) optionally, rinsing of teeth with a solution having a pH of 7 or more before step (v); and
(v) applying the dental care product to a plurality of teeth, wherein the dental care product is preferably applied using an oral irrigator;
wherein steps (i), (ii) and, optionally, (iii) may be carried out sequentially in any order or simultaneously, and wherein step (v) is carried out after the other steps.

In a further aspect, the invention provides a method for remineralising a tooth surface or increasing hardness, e.g. microhardness of a tooth surface, comprising applying said dental care product to a to a plurality of teeth of a subject independent of diagnosis of active caries or demineralisation or tooth erosion.

Self-assembling peptides that are capable of undergoing self-assembly at a pH below 7.5 may also be used for preparation of a dental care product for treatment of a tooth lesion, preferably a caries lesion and/or for remineralising a tooth surface and/or for increasing hardness of teeth.

In another aspect, the invention also relates to a non-therapeutic method for treating teeth, comprising applying a product of the present invention to a plurality of teeth in a subject. Specifically, the invention also provides a non-therapeutic, e.g. a cosmetic, method for treating teeth, e.g. for increasing hardness of tooth surfaces comprising applying a dental care product of the invention to a plurality of teeth. Dental care products of the present invention may also be applied to increase smoothness of tooth surfaces and/or provide increased shine of teeth.

The present invention also discloses kits comprising dental care products of the present invention. Specifically, kits of the present invention may comprise
(a) a dental care product, preferably, in the form of a solution, comprising self-assembling peptides, wherein the self-assembling peptides are capable of undergoing self-assembly at a pH below 7.5, wherein the self-assembling peptides are formulated to maintain monomeric form after application in step (v) for at least 1 minute, wherein said product is suitable for use in step (v) according to any of the preceding claims; and
(b) a solution suitable for use in step (i) and (ii) and, optionally, (iii),
(c) optionally, a solution suitable for use in step (iii) and/or
(d) optionally, a solution suitable for use in step (iv), e.g., water.

In a preferred embodiment, the kit of the present invention comprises solutions (a) and (b), and, if optionally, (c) and/or (d) in one or more container suitable for installation in an oral irrigation device.

The present invention also provides an oral irrigator, comprising at least 2 containers (or tanks) suitable for installing the solutions of said kit, wherein, optionally, the tanks comprise said solutions. Optionally, the oral irrigator comprises at least 3 or at least 4 containers. Preferably, the containers of the kit of the invention fit into said oral irrigator.

In one embodiment, the different solutions for use in the invention are contained in one container (or tank) suitable for installation in an oral irrigation device which allows for sequential administration of said solutions in the sequence (b), optionally, (c), optionally, (d) and then (a) without any need for the subject to exchange containers. The invention also provides an oral irrigator comprising such a tank.

For example, if the outlet of the container for delivery to the oral irrigation device is on the bottom side of the container, the solutions may be in one container in separate compartments, e.g., separated by membranes which, when the bottom solution has been (essentially or completely) used up, are pierced by a suitable device, e.g., comprising one or more thorns, e.g., two thorns, on the bottom of the container, so that the next solution is then delivered to the oral irrigator. Similar results can be achieved by use of suitable valves or seals. For example, the flexible multi-compartment beverage pouches of US7055683B2 or the set for sequential administration of liquids of US4256104A may be adapted for use in the invention.

For example, a container comprising several compartments suitable for installation in an oral irrigation device and suitable for sequential delivery of, firstly, a salt and detergent solution, and then, a solution of the invention comprising self-assembling peptides to said oral irrigation device, is provided. Alternatively, a container comprising several compartments suitable for installation in an oral irrigation device and suitable for sequential delivery of, firstly, a salt and detergent solution, then, an acidic solution, and then, a solution of the invention comprising self-assembling peptides to said oral irrigation device, is provided. Alternatively, a container comprising several compartments suitable for installation in an oral irrigation device and suitable for sequential delivery of, firstly, a salt and detergent solution, then, an acidic solution, then, a solution having a neutral pH and then, a solution of the invention comprising self-assembling peptides to said oral irrigation device, is provided.

Such containers may contain the appropriate solution in the respective compartments. Such containers are preferably for single use. They may be installed in an oral irrigator for use in the method of the invention.

Self-assembling peptides used in the product of the invention undergo self-assembly in response to a certain pH and ionic strength.

Particular self-assembling peptides for use according to the invention are selected such that they are in self-assembled form when the pH of their environment is below a certain pH, e.g. below pH 7.5, e.g., at physiological ionic strength. The pH at which preferred self-assembling peptides of the invention undergo self-assembly is below 7.5, preferably below 7.2, more preferably below 7.0. For example, the pH at which the self-assembling peptide P11-4 (SEQ ID NO:1) and terminally modified P11-4 (SEQ ID NO:2) start to undergo self-assembly is about 7.5. This means that the self-assembling peptides start to self-assemble to a significant extent when the pH drops below 7.5.

As used herein, the pH at which the self-assembling peptide starts to undergo self-assembly refers to the pH below which a significant extent of self-assembly of the peptides in solution is observed, which means that at least about 25%, about 30%, about 35%, about 40%, about 45%, about 50%, about 55%, about 60%, about 65%, about 70%, about 75%, about 80%, about 85%, about 90%, about 95%, about 99% or even about 100% of the peptides found in the dental care product are assembled. In a preferred embodiment, at least about 25% of the peptides found in the dental care product are assembled below the pH at which the peptide starts to undergo self-assembly.

Preferably, at the pH which initiates self-assembly, e.g. about pH 7.5 for P11-4 and modified P11-4, only about 20% or less, preferably only about 15% or less, more preferably 10% or less, and even more preferably 5% or less of the peptides are in a polymeric (or aggregated) state.

In contrast, below the pH which initiates self-assembly, e.g. below pH 7.5 for P11-4 (SEQ ID NO:1) and modified P11-4 (SEQ ID NO:2), a significant extent of self-assembly of the peptides in solution is observed, which means that at least about 25%, about 30%, about 35%, about 40%, about 45%, about 50%, about 55%, about 60%, about 65%, about 70%, about 75%, about 80%, about 85%, about 90%, about 95%, about 99% or even about 100% of the peptides found in the solution are assembled.

As used herein, "self-assembly" of the peptides refers to the spontaneous and reversible organization of peptides with other peptides of their own kind (or peptides having a similar structure) into multimeric assemblies by non-covalent interactions. Non-covalent interactions that are responsible for forming the multimeric assemblies include van-der-Waals, pi-stacking, hydrogen bonds, polar and ionic interactions between the amino acid backbones and/or the amino acid side chains of the peptides.

The self-assembling peptides used in the products of the invention preferably assemble into beta-pleated sheets. In the beta-pleated sheet, the sheet-like structure is created by a series of hydrogen bonds between residues in different polypeptide chains or between residues in different sections of a folded polypeptide. Typically, adjacent polypeptide chains in beta-pleated sheets are anti-parallel, which means that they run in opposite directions. However, the adjacent chains may also run parallel. If several polypeptide chains participate in the sheet formation, the sheet is a rigid wall-like structure. Multiple pleated sheets provide the requisite toughness and rigidity. The peptides that can be used in products of the invention form stable secondary structures upon self-assembly. Preferably, the peptides used in the invention will form long "beta-tapes" comprising a beta-pleated structure of a single molecule in thickness. The peptides may form complex structures during assembly, such as helical tapes (single-molecule thick), twisted ribbons (double tapes), fibrils (twisted stacks of ribbons) and fibers (entwined fibrils). With decreasing pH, helical tapes, twisted ribbons, fibrils and at last fibers may form.

As is known to the skilled person, the assembly state of peptides is also influenced by the ionic strength. The ionic strength of a solution is a function of the concentration of all ions present in that solution. Thus, even at a pH above the pH at which the peptide starts to undergo self-assembly, i.e. when the peptide is substantially monomeric in solution, a particularly high ionic strength might be able to trigger the assembly of the peptide.

However, assembly of the peptides of the present invention is advantageously not triggered when the pH is above 7.5 and ionic strength is in the physiological range, i.e. corresponding to or below the ionic strength corresponding to 150 mM NaCl. The skilled person will know how to determine and measure the ionic strength of a solution. The ionic strength I is generally calculated according to the formula I = ½∑ zᵢ²bᵢ, wherein z is the valence factor and bᵢ is the molality [mol/kg{H₂O}] of the i^{th} ion concentration. The summation, ∑, is taken over all ions in a solution. For example, the ionic strength of a 150 mM NaCl solution is approximately 0.15. This is also approximately the ionic strength of blood. The ionic strength of saliva present in the oral cavity is generally much lower, such as e.g. approximately 0.04. Thus, in a preferred embodiment, the ionic strength in the dental care product of the invention is less than 0.15, less than 0.1, less than 0.05, or less than 0.025. In a preferred embodiment, the ionic strength of the dental care product is less than 0.15. In a further preferred embodiment, the ionic strength is less than 0.1.

The skilled person is aware of numerous methods to determine the ionic strength of a preparation. For example, the ionic strength may be estimated from a measurement of the electric conductance (S = 1/Ω = A/V) of a solution via the Russell's factor as follows: I = 1.6 x 10⁻⁵ x Specific Conductance [µS/cm]. A 150 mM NaCl solution has a conductance of approximately 80-100 mS/cm. Thus, according to the above and the described estimation of the electric conductance, the dental care product will have an electric conductance of below 100 mS/cm, preferably below 80 mS/cm.

Further, the skilled person is aware of numerous methods to determine the pH at which a peptide of the present invention will start self-assembly at a given ionic strength. Suitable methods are denoted e.g. in a publication by Aggeli et al. (2003, J Am Chem Soc, 125, 9619-9628).

The size of the self-assembling peptides used in the products of the invention is not specifically limited. The peptides of the invention may be of any length that allows self-assembly in a pH-dependent manner. Preferably, the peptides will have a size of about 4-200 amino acids, more preferably, 6-50 amino acids, 8-30 amino acids, 9-20 amino acids or 10-11 amino acids. In a particularly preferred embodiment, the self-assembling peptides have a length of 11 amino acids.

The self-assembling peptides may be prepared by any suitable method that is commonly known in the field of peptide synthesis. For example, peptides with a length of more than 50 amino acids may be prepared by recombinant methods. In one embodiment, the self-assembling peptides are produced as fusion peptides. As used herein, a fusion peptide refers to a fusion of a first amino acid sequence comprising the self-assembling peptide of interest which is N-terminally or C-terminally linked to a second amino acid sequence. The second amino acid sequence may be an affinity tag, i.e. an amino acid sequence that is fused to the C-, or N-terminal of the self-assembling peptide and which exhibits an increased affinity to another compound, thereby allowing purification of the fusion peptide. Preferably, the tag sequence is removed from the self-assembling peptide of interest after purification, for example by providing a proteolytic cleavage site between the self-assembling peptide and the affinity tag. In one embodiment, the self-assembling peptide is prepared as disclosed in Kyle et al., 2010, Biomaterials 31, 9395-9405 and Kyle et al. 2009, Trends in Biotechnol. 27 (7), 423-433.

Smaller self-assembling peptides are usually prepared by chemical synthesis. For example, the peptides may be chemically synthesized by solid phase or liquid phase methods. Protocols for solution-phase chemical synthesis of peptides have been described (see, for example, Andersson et al., Biopolymers 55:227-250, 2000). For solid phase synthesis the technique described by Merrifield (J. Am. Chem. Soc., 1964, 85, 2149-2154) may be used. In this approach, the growing peptide is anchored on an insoluble resin, and unreacted soluble reagents are removed by filtration or washing steps without manipulative losses. Solid phase peptide synthesis can be readily performed by use of automated devices.

The peptides used in the products of the invention may comprise any natural, proteinogenic amino acid. In addition, the peptides may also comprise unusual, non-proteinogenic amino acids, such as carnitine, gamma-aminobutyric acid (GABA), hydroxyproline, selenomethionine, hypusine, lanthionine, 2-aminoisobutyric acid, dehydroalanine, ornithine (Orn, O), citrulline, beta alanine (3-aminopropanoic acid), and the like. Non-proteinogenic amino acids can be incorporated into the peptide by post-translational modification or by direct incorporation during chemical synthesis of the peptide.

The peptides preferably comprise amino acid side chains that include a -COOH group. Amino acid side chains with a -COOH will be deprotonated at pH values above their nominal pK values. For example, amino acids which comprise a -COOH group in their side chain such as aspartic acid (Asp, D) and glutamic acid (Glu, E) are essentially deprotonated at a pH above neutral, i.e. at pH 7, because they exhibit a low pKa (Asp: 3.71; Glu: 4.15). In the self-assembling peptides used in the products of the present invention, the amino acid side chains containing a -COOH group are specifically located in the peptide chain so as to control the electrostatic interactions between neighbouring peptides, i.e. so that adjacent, identical, self-assembling peptides are repelled through electrostatic interactions when the -COOH group is deprotonated to -COO-, and to dominate the association free energy in bonds between peptides. Reducing the pH below a certain threshold, i.e. the pH at which the peptide starts to undergo self-assembly, such as about pH 7.5 for P11-4 (SEQ ID NO:1) and modified P11-4 (SEQ ID NO:2), leads to protonation of the -COOH group in the self-assembling peptides of the present invention which reduces the repelling electrostatic interactions between the peptides and allows self-assembly of the peptides.

The peptides used in the products of the invention preferably comprise the sequence of the formula X1-X2-X1-X2-X1, wherein X1 is an amino acid with an acidic side chain, and X2 is an amino acid with a hydrophobic side chain selected from the group consisting of alanine, valine, isoleucine, leucine, methionine, phenylalanine, tyrosine, and tryptophan (SEQ ID NO: 3).

In a more preferred embodiment, the self-assembling peptides used in the products of the invention comprise the sequence Glu-X2-Glu-X2-Glu, wherein X2 is an amino acid with a hydrophobic side chain selected from the group consisting of alanine, valine, isoleucine, leucine, methionine, phenylalanine, tyrosine, and tryptophan (SEQ ID NO: 4) or Asp-X2-Asp-X2-Asp, wherein X2 is an amino acid with a hydrophobic side chain selected from the group consisting of alanine, valine, isoleucine, leucine, methionine, phenylalanine, tyrosine, and tryptophan (SEQ ID NO:5).

In another preferred embodiment, the self-assembling peptides used in the products of the present invention comprise or consist of the sequence Gln-Gln-Arg-Phe-Glu-Trp-Glu-Phe-Glu-Gln-Gln (P11-4, SEQ ID NO:1), or a sequence having at least 40 %, at least 50%, at least 80%, or at least 90% sequence identity thereto. Modified P11-4 as shown in SEQ ID NO:2 may also be used.

For the peptides referred to herein as P11-4, the switch from the monomeric to the assembled, multimeric form is mainly controlled by the pH in the use of the invention. If the pH is below pH 7.5, the peptide assembles. If the pH is higher, the state of the peptide is essentially monomeric.

The peptide having at least 40%, preferably, at least 80% or more sequence identity to SEQ ID NO:1 preferably comprises glutamic acid, or aspartic acid at positions which correspond to amino acids 5, 7 and 9 of SEQ ID NO:1 or 2. Specifically, the peptide sequence having at least 80% or more sequence identity to SEQ ID NO:1 preferably comprises glutamic acid at positions which correspond to amino acids 5, 7 and 9 of SEQ ID NO:1. Preferably, the remaining amino acid positions are amino acids with a hydrophobic side chain selected from the group consisting of alanine, valine, isoleucine, leucine, methionine, phenylalanine, tyrosine, and tryptophan. Preferably, the remaining amino acid positions are not amino acids that have basic side chains, i.e. amino acids that would be positively charged at a pH around neutral.

In one embodiment, the peptides used in the products of the invention comprise or consist of sequences that differ from those depicted in SEQ ID NOs:1 and 2 by the replacement of 1, 2 or 3 amino acids. Generally, each of the amino acid residues within the peptide sequence of SEQ ID NOs:1 and 2 may be substituted by another residue, as long as the resulting peptide is still capable of undergoing self-assembly at a pH value below 7.5. It is preferred that the substitutions are conservative substitutions, i.e. substitutions of one or more amino acid residues by an amino acid of a similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine and lysine. Examples of charged polar (acidic) amino acids comprise aspartic acid and glutamic acid.

Further, the peptides used in the products of the invention may be structurally modified in one or more amino acid positions, e.g. by the introduction of one or more modified amino acids. According to the invention, these modified amino acids may be amino acids that have been changed by e.g. biotinylation, phosphorylation, glycosylation, acetylation, branching and/or cyclization. Further, the peptides of the invention may additionally or alternatively contain other modifications, such as terminal blocking groups, formyl-, gamma-carboxyglutamic acid hydroxyl-, methyl-, phosphoryl-, pyrrolidone carboxylic acid-, and/or sulphate-groups. In a preferred embodiment, the peptides of the invention are acetylated at their N-terminus and/or amidated, e.g. with an NH₂-group, at their C-terminal end. A particularly preferred embodiment is a peptide P11-4 that is N-terminally acetylated and C-terminally amidated with a NH₂-group, as depicted in the following sequence: CH₃CO-QQRFEWEFEQQ-NH₂ (SEQ ID NO:2).

In the dental care products of the present invention, self-assembling peptides are applied to tooth surfaces in a monomeric state, in which they are capable of diffusing into tooth lesions where remineralisation is to be achieved.

The average pH of saliva of healthy human subjects was found to be about 7.06 ± 0.04, for subjects with chronic gingivitis, 7.24 ± 0.10 while average pH of those having chronic generalized periodontitis was 6.85 ± 0.11 (Baliga et al., 2013. J Indian Soc Periodontol. 17:461-465), wherein the pH is well-buffered, minimizing, e.g., erosion of teeth by acidic food or drink.

In a tooth lesion, the pH is normally between 5.0 and 6.5 or lower as a result from the continuous production of lactic acid by lactic acid bacteria which form the microflora of the oral cavity (Murakami et al., 2006. Dent Mater J 25(3):423-429). As an early caries lesion, e.g. a subsurface caries lesion, has acid conditions and high ionic strength, the self-assembling peptides will start to assemble *in situ*, forming a three dimensional network. Thus, the pH-induced assembly of the monomeric peptides starts within the lesion, thereby forming multimeric assemblies which can act as scaffolds for subsequent calcium phosphate deposition. This process is also referred to in the context of the invention as polymerisation. After network formation, calcium is attracted from the saliva, generating nucleation islands for the formation of calcium phosphate crystals (hydroxyapatite) in the early caries lesion. Further, the surface area of cavitated caries is very high including deep pits and grooves.

Application of the self-assembling peptides in their monomeric, not-assembled state is particularly critical for treatment of subsurface caries lesions, since the formed scaffolds are too large to diffuse through pores. In order to ensure a sufficient delivery of peptides to the lesion, their assembly has to be prevented until they reach their site of action.

Thus, maintenance of a pH above the pH at which the peptides start to undergo self-assembly is required, preferably until a sufficient amount of peptide monomers is delivered (e.g., by diffusion) to the site of action, e.g. a tooth lesion. In the dental care products of the present invention, effective mono-merisation may be achieved by using a product having a pH which is more than 0.5 pH units above the pH at which the peptides start to undergo self-assembly. Preferably, the pH in the products of the invention is more than 0.6 pH units above the pH at which the peptides start to undergo self-assembly. Even more preferably, the pH is more than 0.7 pH units above the pH at which the peptides start to undergo self-assembly.

For example, when using the peptides referred to herein as SEQ ID NO:1 or SEQ ID NO:2, the product may have a pH of 8.0 or higher, since self-assembly of these peptides starts at pH 7.5. Thus, in the product of the invention, the pH may be 7.5 - 9.0. In a preferred embodiment the pH in the product is 7.8 - 8.5, more preferably, 8.0 - 8.2.

The dental care product typically comprises one or more typical ingredients of the respective dental care product, e.g. typical pharmaceutically acceptable bases having the required pH, wherein the self-assembling peptides may be incorporated, e.g., in water or a buffer solution, e.g., as described herein.

The pH of the dental care product of the invention may be buffered to ensure that a substantial percentage of the self-assembling peptide stays in monomeric form for a sufficient time to ensure that it reaches the sites of potential lesions.

Suitable buffers include TAPS ({[tris(hydroxymethyl)methyl] amino}propanesulfonic acid), Bicine (N,N-bis (2-hydroxyethyl) glycine), TRIS (Tris(hydroxymethyl)-aminomethan), Tricine (N-tris(hydroxymethyl)methylglycine), TAPSO (3-[N-Tris(hydroxyl-methyl)methylamino]-2-hydroxypropanesulfonic acid), HEPES (4-2-hydroxy-ethyl-1-piperazineethanesulfonic acid), TES (2-{[tris (hydroxy-methyl)methyl]amino}ethanesulfonic acid), and other buffers that maintain a similar pH range. Acid buffers comprising, e.g., citric acid, phosphoric acid, and others may also be used in conjunction with any of the above buffers and/or an alkaline buffer comprising, e.g., sodium hydroxide, potassium hydroxide, ammonium hydroxide, sodium phosphate di- and tri-basic, potassium phosphate di- and tri-basic, sodium tripolyphosphate, TRIS, triethanolamine, polyethylenimine, to obtain the desired pH which is more than 0.5 pH units above the pH at which the peptide starts to undergo self-assembly and to provide buffering capacity. In a preferred embodiment, the buffer to provide a specific pH which is more than 0.5 pH units above the pH at which the peptide starts to undergo self-assembly, such as pH 7.5, is TRIS.

To improve taste and acceptance of the products, dental care products of the invention may comprise sugar and/or sugar substitutes, which, preferably, do not promote tooth decay, e.g., polyols or sugar alcohols such as sorbitol, mannitol, maltitol, lactitol, isomalt, xylitol and/or erythritol, or D-tagatose and/or trehalose. In one embodiment, the products are sugar-free products, i.e., they do not comprise sucrose or glucose in significant amounts, or not at all.

It is particularly advantageous if the dental care product, in addition to the self-assembling peptides, comprises xylitol (D-xylit), which has been shown to be anti-cariogenic and helpful for remineralization of teeth. It also reduces biofilm and plaque and thus facilitates access of the self-assembling peptides of the invention to the tooth surface and potential lesions. Of course, xylitol is not used for application in dogs, cows, goats, rabbits or other animals for which the substance is toxic. Xylitol can be used in products of the invention for use in human subjects or e.g., cats, preferably, human subjects.

Suitable solutions comprising self-assembling peptides may e.g. comprise 1-10% PVP, 0-30% glycerol, preferably, 1-10% glycerol, 1-10% of a sugar substitute, e.g. sucarose or xylitol, 0.01-1% TRIS, 0.1-1% NaOH, 0.1-1% self-assembling peptide (e.g., P11-4 having SEQ ID NO: 1), and water ad 100. % is w/w. Preferably, the solution has a viscosity suitable for use with an oral irrigator.

WO 2014/027012 describes production of a composition comprising self-assembling peptides which has a pH 0.1 to 0.5 pH units above the pH at which the peptides starts to undergo self-assembly. Said compositions are intended for professional application directly to caries lesions. Although the methods disclosed in WO 2014/027012 may be used or adapted for the present invention, the dental care product of the present invention preferably differs from the product produced according to WO 2014/027012, and from the intermediate product of the process disclosed therein comprising a volatile amount which increases the pH.

In one embodiment, the dental care product of the invention does not comprise mineral particles.

At the high pH of the dental care product of the invention, before application of the product in step (v), at least 70%, preferably at least 80%, more preferably at least 90% or at least 95% or at least 99% of the self-assembling peptides are present in a monomeric, non-assembled state in the product.

Upon application to the plurality of teeth in the oral cavity in step (v), at least 40%, at least 50%, at least 60%, preferably, at least 70% of the peptides remain in a monomeric form for at least 1 minute. Preferably, said percentage of peptides remains in a monomeric state for at least 2 minutes, at least 3 minutes, at least 5 minutes or at least 10 minutes after application.

In this way, the method of the present invention allows the effective, non-targeted delivery of monomeric peptides present in a monomeric state to a plurality of teeth, preferably to all teeth. The peptides form assemblies, and scaffolds for remineralization only at their site of action, wherein the most preferred sites of action are lesions such as caries lesions. After distribution on the surface of the teeth, a polymeric or assembled film of self-assembling peptides may also form and lead to increased mineralization, and, accordingly, hardness of the tooth surface.

In a particularly preferred embodiment, the dental care product comprises the peptide P11-4 (SEQ ID NO:1) or modified P11-4 as shown in SEQ ID NO:2 and the product has a pH about 8.0 or more, wherein, preferably, the pH is buffered at about 8.0 or more.

In one embodiment, at least 70% of the peptides are present in a monomeric state in the product before application to a plurality of teeth in step (v), wherein at least 50%, preferably at least 60%, more preferably at least 70% of the peptides are present in a monomeric state for at least 1 minute, preferably at least 2 minutes, more preferably at least 3 minutes or at least 5 minutes, after application.

In another embodiment, at least 80% of the peptides are present in a monomeric state in the product before application to a plurality of teeth in step (v), wherein at least 50%, at least 60%, at least 70% or at least 80% of the peptides are present in a monomeric state for at least 1 minute, preferably at least 2 minutes, more preferably at least 3 minutes or at least 5 minutes, after application.

In a particularly preferred embodiment, at least 90% of the peptides are present in a monomeric state in the product before application to a plurality of teeth in step (v), wherein at least 50%, at least 60%, at least 70%, at least 80% or at least 90% of the peptides are present in a monomeric state for at least 1 minute, preferably at least 2 minutes, more preferably at least 3 minutes, after application.

Preferably, at least 90% of the self-assembling peptide are present in monomeric state in the dental care product before application.

Preferably at least 60%, of the self-assembling peptide are present in monomeric state for at least 1 minute, preferably at least 2 minutes, more preferably at least 3 minutes after application to a plurality of teeth in step (v).

The skilled person will be able to determine whether essentially all of the self-assembling peptides are in a monomeric form by means of routine experimentation. For example, the assembly state of the peptides in solution can be determined by nuclear magnetic resonance (NMR), such as ¹H-NMR, by circular dichroism analysis, by dynamic light scattering (DLS) analysis, diffusing-wave spectroscopy, native electrophoretic methods, viscosity measurements (rheology), Quartz crystal microbalance with dissipation monitoring (QCMD) and the like, preferably, by native electrophoretic methods.

The products of the present invention are particularly advantageous, because the loss of significant amounts of peptides due to self-assembly of the peptides outside of the sites of action is avoided. At the same time the products of the present invention facilitate distribution of the necessary concentration of monomeric self-assembling peptides on the teeth, and, if applicable, inside the lesions to enable self-assembly.

To prevent degradation and/or precipitation of the peptides in solution the pH will normally not be increased to a value higher than 10.5. It is commonly known that the chemical properties of a peptide depend on the amino acid sequence. For example, reversible oxidation of cysteine and methionine residues may be accelerated at higher pH, where the thiol is more easily deprotonated and readily forms intra-chain or inter-chain disulfide bonds. The skilled person will be aware of amino acid side chains that are detrimentally affected by a basic pH and be able to determine the maximal pH that keeps the integrity of the self-assembling peptide intact by routine experimentation. For example, this maximal pH may be predicted on the basis of the known properties of each amino acid present in the peptide. Alternatively, biochemical methods, such as electrophoretic methods, may be employed to determine the integrity of the peptide.

It is known to the skilled person that the peptide concentration may influence the assembly of peptides, i.e. a particularly high peptide concentration may trigger assembly ahead of time. Further, an exceptionally low peptide concentration may prevent assembly of the peptides of the invention, i.e. even under low pH conditions as present in tooth lesions and the oral cavity.

The peptide concentration in the dental care product of the invention may be between 1 to 50000 mg peptide/kg bulk product, 100 to 30000 mg peptide/kg bulk product, 200 to 10000 mg peptide /kg bulk product, 500-5000 or 1000 to 2000 mg peptide/kg bulk product, preferably 200-1000 mg/kg.

In one embodiment, the dental care product comprises capsules comprising self-assembling peptides. This enhances storage stability of the peptides, in particular, in solutions which may otherwise, upon storage lead to aggregation of the self-assembling peptides. Such capsules may set the peptide free after mechanical stress such as application with an oral irrigator.

Suitable methods of encapsulation are provided, e.g. in Nedovic et al, Procedia Food Science 1, 2011, 1806-1815. Preferably, self-assembling peptides of the present invention may be encapsulated by spray drying, extrusion methods, e.g. for alginates as shell material, emulsification, or fluid bed coating on calcium phosphate particles. For example, alginate capsules comprising self-assembling peptide P11-4 may be used.

Suitable encapsulation shells for self-assembling peptides may comprise starch and derivatives, e.g. amylose, amylopectin, dextrins, maltodextrins, polydextrose, syrups, cellulose and derivatives, plant exudates and extracts, e.g. gum Arabic, gum tragacanth, gum karaya, mesquite gum, galactomannans, pectins and soluble soybean polysaccharides, marine extracts, e.g. carrageenans and alginate, microbial and animal polysaccharides, e.g. dextran, chitosan, xanthan and gellan, proteins, e.g. milk and whey proteins such as caseins, gelatine and gluten, lipid materials suitable for food applications, e.g. fatty acids and fatty alcohols, waxes such as beeswax, carnauba wax and candellia wax, glycerides and phospholipids, or other materials such as PVP, paraffin, shellac, inorganic materials.

Liquid dental care products typically used for dental hygiene such as mouthwash, mouth spray or solutions, e.g., suitable for use in an oral irrigator, are particularly useful in the invention.

Dental care products of the invention are effective in treating or preventing a tooth lesion, and/or in remineralising a tooth surface and/or in increasing hardness, e.g. microhardness, of a tooth surface in a subject after administration on a regular basis. In this context, administration on a regular basis comprises administration every 2 to 6 months, at least every 2 months, at least every month, at least every week or daily. For example, microhardness, e.g., microhardness of demineralised teeth, may be increased by at least 10%, preferably, by at least 20% upon regular administration every two months for one year. Microhardness, e.g, Vickers hardness or Knoop hardness, preferably, Knoop hardness, may be measured, e.g., by methods described in Chuenarron et al. (2009, Materials Research 12(4), 473-476), wherein the indentation load preferably is 100 g.

The following examples are intended to illustrate, but not to limit the invention. All references cited herein are herewith fully incorporated.

### BRIEF DESCRIPTION OF THE FIGURES

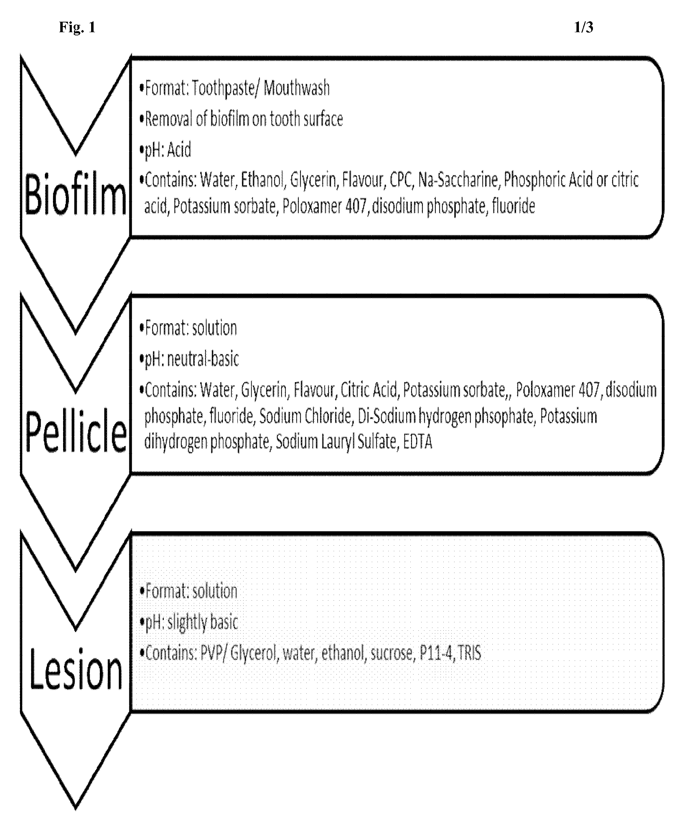

Fig. 1 shows a flow scheme of an exemplary treatment of the invention

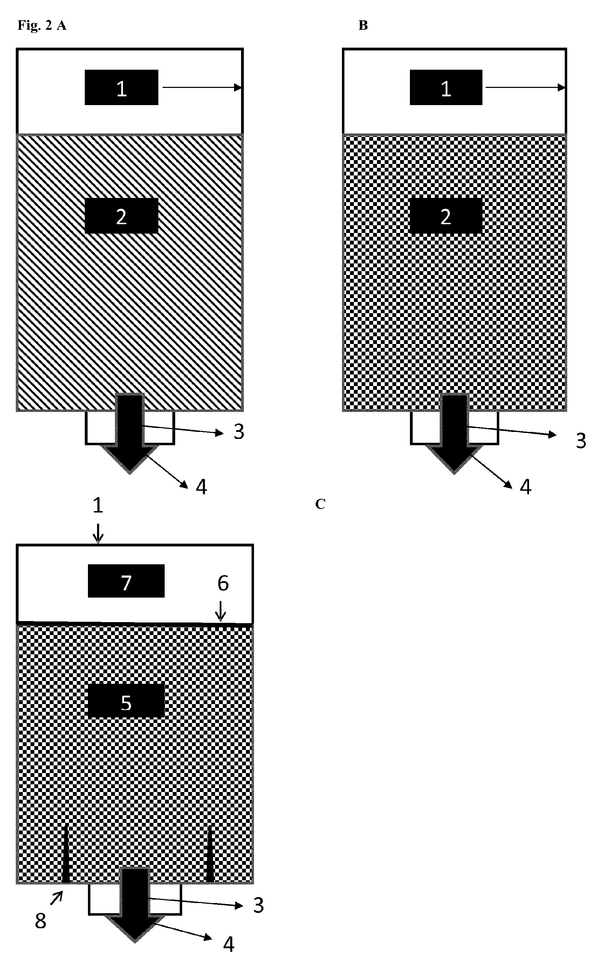

Fig. 2 shows exemplary containers for use in the invention. **A**, **B** show a set of single use cartridges (containers) for oral irrigator. C shows a single cartridge comprising two compartments. (1) container; (2) in 2A, acidic pellicle removing solution; in 2B, solution of the invention comprising self-assembling peptide; (3) adaptor to oral irrigation unit; (4) direction of flow; (5) compartment comprising acidic pellicle removing solution; (6) membrane; (7) compartment comprising solution of the invention comprising self-assembling peptide; (8) thorns.

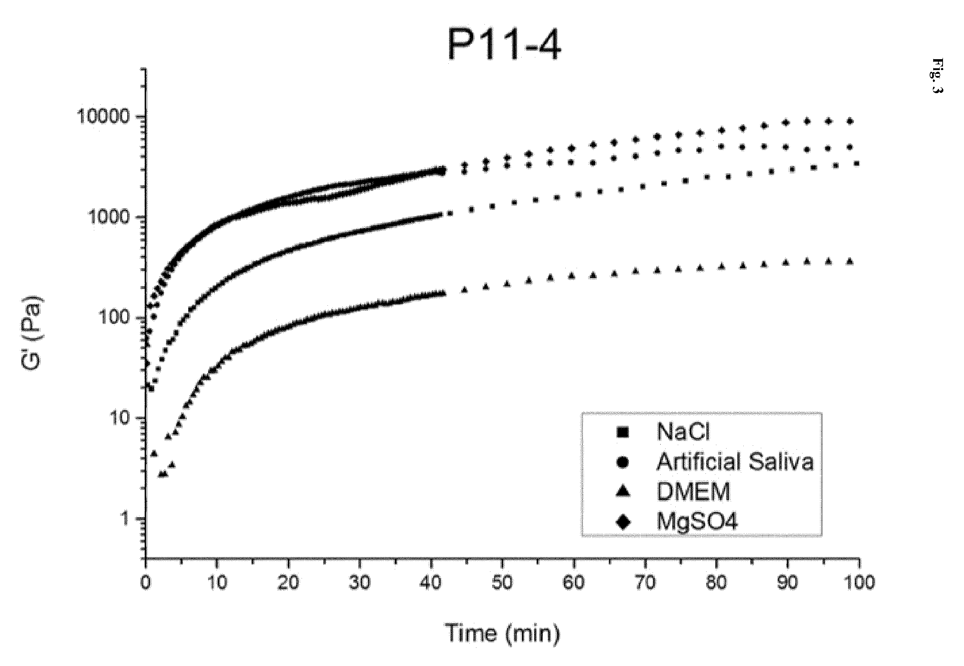

**Fig. 3** shows self-assembling kinetic of P11-4, assessed by measurements of elastic modulus G'. P11-4 was prepared at 15 mg/ml with different buffer compositions (see below) measured at 0.1% strain, 37°C and a frequency of 1 rad/second. Self-assembly of peptide monomers to polymers takes more than 5 min independent of the used ionic strength at neutral pH.
   Squares (top line of symbols) - NaCl: Tris (0.055 M) + NaCl, final ionic strength 142 mM;
   Artifical saliva (second line of symbols from top): Tris, Ca(NO₃)₂, KH₂PO₄ (ratio 0.77:0.14: 0.08), ionic strength 142 mM;
   Dulbecco's Modified Eagle Medium (DMEM): NaCl, NaHCO₃,KCl,CaCl₂, MgSO₄ (ratio 0.66:0.4:0.05: 0.02:0.007), ionic strength 165 mM;
   MgSO₄: 0.055 M Tris + 0.192 M MgSO₄ 0.14 M Ionic strength

### EXAMPLES

### Example 1: Exemplary solutions for use in the invention

### Base Fomulation Solution I

| Ingredient | % (w/w) |
|---|---|
| Glycerol | 1-30% |
| Flavour | 0.1-2 |
| Citric acid | 0.05-1 |
| Potassium sorbate | 0.05-0.2 |
| Poloxamer 407 | 0.1-1 |
| Natrium fluoride | 0.1-1 |
| Sodium Chloride | 0.5-1.0 |
| Di-Sodium hydrogen phosphate | 0.1-1 |
| Potassium chloride | 0.001-0.1 |
| Potassium dihydrogen phosphate | 0.01-0.1 |
| Surfactant | 0.1-2 |
| Water | ad 100 |

### Formulation for Solution II

| Ingredient | % (w/w) |
|---|---|
| PVP | 1-10 |
| Glycerol | 1-30 |
| Sucarose | 1-10% |
| TRIS | 0.01-1 |
| Sodium Hydroxyde | 0.01-1 |
| P11-4 | 0.001-1 |
| Water | ad 100 |

### Example 2: Exemplary treatment methods of the invention

### Mouthwash

### Step A: removal of biofilm

- Subject cleans teeth with toothpaste, e.g., abrasive toothpaste
- spit out remnants of tooth paste

### Step B: removal of pellicle

- Subject takes 5 ml of mouthwash IA comprising Water, Glycerol, Flavour, Citric acid, Potassium sorbate, Poloxamer 407, sodium fluoride, Sodium chloride, Di-Sodium hydrogen phsophate, Potassium chloride, Potassium dihydrogen phosphate, EDTA (concentrations cf. Example I);
- or, alternatively, Subject takes 5 ml of mouthwash IB comprising Water, Glycerol, Flavour, Citric Acid, Potassium sorbate, Poloxamer 407, sodium fluoride, Sodium chloride, Di-Sodium hydrogen phsophate, Potassium chloride, Potassium dihydrogen phosphate, Sodium Lauryl Sulfate SDS (concentrations cf. Example I);
- or, alternatively, Subject takes 5 ml of mouthwash IC comprising Water, Glycerol, Flavour, Citric Acid, Potassium sorbate, Poloxamer 407, sodium fluoride, Sodium Chloride, Di-Sodium hydrogen phsophate, Potassium Chloride, Potassium dihydrogen phosphate, Cocoam-idopropylbetaine (concentrations cf. Example I)
   and swirls the Mouthwash for about 5 min in the oral cavity
- Subject spits out the mouthwash

### Step C: Regeneration

- Subject takes 5 ml of mouthwash II comprising PVP/ Glycerol, water, ethanol, sucrose, P11-4, Sodium hydroxide, TRIS (concentrations cf. Example I) and swirls the solution for about 5 min in the oral cavity
- Subject spits out the mouthwash after 5 min.

### Oral irrigator

### Step A: removal of biofilm

- Subject cleans teeth with toothpaste, e.g., abrasive toothpaste
- spit out remnants of tooth paste

### Step B: removal of pellicle

- Subject places cartridge I on oral irrigator. Cartridge I contains 300 ml-600 ml acidic solution (pH 4-7) comprising:
   - Solution IA: Water, Glycerol, Flavour, Citric acid, Potassium sorbate, Poloxamer 407, sodium fluoride, Sodium chloride, Di-Sodium hydrogen phosphate, Potassium chloride, Potassium dihydrogen phosphate, EDTA (concentrations cf. Example I);
   - or, alternatively Solution IB comprising Water, Glycerol, Flavour, Citric Acid, Potassium sorbate, Poloxamer 407, sodium fluoride, Sodium chloride, Di-Sodium hydrogen phosphate, Potassium chloride, Potassium dihydrogen phosphate, Sodium Lauryl Sulfate SDS (concentrations cf. Example I);
   - or, alternatively, Solution IC comprising Water, Glycerol, Flavour, Citric Acid, Potassium sorbate, Poloxamer 407, sodium fluoride, Sodium Chloride, Di-Sodium hydrogen phosphate, Potassium Chloride, Potassium dihydrogen phosphate, Cocoami-dopropylbetaine (concentrations cf. Example I)
- Subject starts cleaning the teeth with the oral irrigator until cartridge I is finished. During the procedure, subject spits out the surplus fluid.

### Step C: Regeneration

- Subject removes cartridge I and places cartridge II on oral irrigator. Cartridge II contains 100 ml of a slightly basic ( pH > 7.5 ) solution comprising PVP/ Glycerol, water, ethanol, sucrose, P11-4, Sodium Hydroxide, TRIS solution (concentrations cf. Example I).
- Subject starts cleaning the teeth with the oral irrigator until cartridge II is finished. During the procedure, subject spits out the surplus fluid.

## Claims

1. A dental care product for use in a method of treating or preventing a tooth lesion and/or in remineralising a tooth surface, wherein the dental care product comprises self-assembling peptides and wherein the self-assembling peptides maintain monomeric form after application in step (v) for at least 1 minute, wherein the self-assembling peptides are capable of undergoing self-assembly at a pH below 7.5, wherein the method comprises
(i) removing dental biofilm from a plurality of teeth by a method selected from the group comprising tooth brushing, flossing of teeth, use of an oral irrigator, and rinsing with a salt and detergent solution;
(ii) removing dental pellicle from a plurality of teeth by a method selected from the group comprising tooth brushing, flossing of teeth, use of an oral irrigator, and rinsing with a salt and detergent solution;
(iii) optionally, removing inorganic deposit from a plurality of teeth by a method selected from the group comprising tooth brushing, rinsing with an acidic solution or rinsing with a salt and detergent solution, optionally, using an oral irrigator, and;
(iv) optionally, rinsing of teeth with a solution having a pH of 7 or more before step (v); and
(v) applying the dental care product to a plurality of teeth, wherein the dental care product is preferably applied using an oral irrigator;
wherein steps (i), (ii) and, optionally, (iii) may be carried out sequentially in any order or simultaneously, and wherein step (v) is carried out after the other steps.

2. The dental care product for use according to claim 1, wherein steps (i), (ii) and (v), and, optionally, (iii) and/or (iv), are carried out using an oral irrigator.

3. The dental care product for use according to any of the preceding claims, wherein the method comprises tooth brushing followed by using an oral irrigator, wherein the oral irrigator is at least used for step (v), wherein preferably, the oral irrigator is also used for steps (i), (ii), and, optionally, (iii) and/or (iv).

4. The dental care product for use according to any of the preceding claims, wherein use of an oral irrigator in steps (i), (ii) and, optionally, (iii) involves oral irrigation with a solution comprising water, salt and detergent.

5. The dental care product for use according to any of the preceding claims, wherein the dental care product is for use independent of prior diagnosis of active caries.

6. The dental care product for use according to any of the preceding claims, wherein the dental care product is suitable for home application, wherein it is used without prior etching or NaOCl treatment.

7. The dental care product for use according to any of the preceding claims for use in treating a tooth lesion, preferably a caries lesion.

8. The dental care product for use according to any of the preceding claims for use in remineralising a tooth surface.

9. The dental care product for use according to any of the preceding claims, wherein the pH of the dental care product is 7.5 - 9.0, preferably, 7.8 - 8.5, more preferably, 8.0 - 8.2.

10. The dental care product for use according to any of the preceding claims, wherein the pH of the solution is more than 0.5 pH units above the pH at which the peptides start to undergo self-assembly.

11. The dental care product according to any of the preceding claims, wherein the concentration of the self-assembling peptides is 1 - 50000 mg/kg.

12. The dental care product for use according to any of the preceding claims, wherein said self-assembling peptides comprise the sequence of SEQ ID NO: 3, wherein said peptides preferably comprise the sequence of any one of SEQ ID NOs: 4 or 5.

13. The dental care product for use according to any of the preceding claims, wherein said self-assembling peptides comprise a sequence having at least 80% sequence identity to one of the sequences of SEQ ID NOs: 1 or 2, wherein said peptides preferably comprise the sequence of SEQ ID NO: 1.

14. The dental care product for use according to any of the preceding claims, wherein the product is applied at least every 6 months, preferably, at least every 2 months or at least every month.

15. A non-therapeutic method for treating teeth, comprising applying a product comprising self-assembling peptides to a plurality of teeth and wherein the self-assembling peptides maintain monomeric form after application in step (v) for at least 1 minute, wherein the self-assembling peptides are capable of undergoing self-assembly at a pH below 7.5, wherein the method comprises
(i) removing dental biofilm from a plurality of teeth by a method selected from the group comprising tooth brushing, flossing of teeth, use of an oral irrigator, and rinsing with a salt and detergent solution;
(ii) removing dental pellicle from a plurality of teeth by a method selected from the group comprising tooth brushing, flossing of teeth, use of an oral irrigator, and rinsing with a salt and detergent solution;
(iii) optionally, removing inorganic deposit from a plurality of teeth by a method selected from the group comprising tooth brushing, rinsing with an acidic solution or rinsing with a salt and detergent solution, optionally, using an oral irrigator, and;
(iv) optionally, rinsing of teeth with a solution having a pH of 7 or more before step (v); and
(v) applying the dental care product to a plurality of teeth, wherein the dental care product is preferably applied using an oral irrigator;
wherein steps (i), (ii), and optionally, (iii) may be carried out sequentially in any order or simultaneously, and wherein step (v) is carried out after the other steps.

16. A kit comprising
(a) a dental care product comprising self-assembling peptides, wherein the self-assembling peptides maintain monomeric form after application in step (v) for at least 1 minute, wherein the self-assembling peptides are capable of undergoing self-assembly at a pH below 7.5, suitable for use in step (v) according to any of the preceding claims; and
(b) a solution suitable for use in step (i) and (ii) according to any of the preceding claims,
(c) optionally, a solution suitable for use in step (iii) according to any of the preceding claims and/or
(d) optionally, a solution suitable for use in step (iv) according to any of the preceding claims.

17. An oral irrigator suitable for carrying out the method according to any of claim 1-15, wherein the oral irrigator comprises at least 2 tanks suitable for holding the solutions of the kit of claim 16 or one tank comprising at least 2 compartments suitable for sequential delivery of said solutions, wherein, optionally, the tank(s) comprise said solutions.


SEQUENCE LISTING

<110> Credentis AG

<120> Method and product for caries treatment

<130> CRE16963EP

<160> 5

<170> BiSSAP 1.3

<210> 1
<211> 11
<212> PRT
<213> Artificial Sequence

<220>
<223> designed self-assembling peptide P11-4

<400> 1
Gln Gln Arg Phe Glu Trp Glu Phe Glu Gln Gln
1               5                   10

<210> 2
<211> 11
<212> PRT
<213> Artificial Sequence

<220>
<221> MOD_RES
<222> 1..1
<223> ACETYLATION

<220>
<223> designed self-assembling peptide P11-4mod

<220>
<221> MOD_RES
<222> 11..11
<223> AMIDATION

<400> 2
Gln Gln Arg Phe Glu Trp Glu Phe Glu Gln Gln
1               5                   10

<210> 3
<211> 5
<212> PRT
<213> Artificial Sequence

<220>
<223> designed self-assembling peptide

<220>
<221> VARIANT
<222> 1..1
<223> amino acid with acidic side chain

<220>
<221> VARIANT
<222> 2..2
<223> amino acid with hydrophobic side chain that is selected from the

group consisting of alanine, valine, isoleucine, leucine,
      methionine, phenylalanine, tyrosine and tryptophan

<220>
<221> VARIANT
<222> 3..3
<223> amino acid with acidic side chain

<220>
<221> VARIANT
<222> 4..4
<223> amino acid with hydrophobic side chain that is selected from the
      group consisting of alanine, valine, isoleucine, leucine,
      methionine, phenylalanine, tyrosine and tryptophan

<220>
<221> VARIANT
<222> 5..5
<223> amino acid with acidic side chain

<400> 3
Xaa Xaa Xaa Xaa Xaa
1               5

<210> 4
<211> 5
<212> PRT
<213> Artificial Sequence

<220>
<223> designed self-assembling peptide

<220>
<221> VARIANT
<222> 2..2
<223> amino acid with hydrophobic side chain that is selected from the
      group consisting of alanine, valine, isoleucine, leucine,
      methionine, phenylalanine, tyrosine and tryptophan

<220>
<221> VARIANT
<222> 4..4
<223> amino acid with hydrophobic side chain that is selected from the
      group consisting of alanine, valine, isoleucine, leucine,
      methionine, phenylalanine, tyrosine and tryptophan

<400> 4
Glu Xaa Glu Xaa Glu
1               5

<210> 5
<211> 5
<212> PRT
<213> Artificial Sequence

<220>
<223> designed self-assembling peptide

<220>
<221> VARIANT
<222> 2..2
<223> amino acid with hydrophobic side chain that is selected from the
      group consisting of alanine, valine, isoleucine, leucine,

      methionine, phenylalanine, tyrosine and tryptophan

<220>
<221> VARIANT
<222> 4..4
<223> amino acid with hydrophobic side chain that is selected from the
      group consisting of alanine, valine, isoleucine, leucine,
      methionine, phenylalanine, tyrosine and tryptophan

<400> 5
Asp Xaa Asp Xaa Asp
1               5